# EUROPEAN PATENT SPECIFICATION

(11) **EP 3 978 007 B1**
(45) Date of publication and mention of the grant of the patent: **13.03.2024**
(21) Application number: 20815527.5
(22) Date of filing: 14.05.2020
(51) Int. Cl.: A61K 31/352, A61K 31/05, A61K 36/185, A61Q 11/00, A61Q 13/00, A61K 8/34, A61K 8/49, A61K 8/9789, A61K 47/00, A61K 47/69, A61K 8/73

(54) **INCLUSION COMPOUND CONTAINING NON-PSYCHOACTIVE CANNABINOID AND METHOD FOR PREPARATION THEREOF**
EINSCHLUSSVERBINDUNG MIT NICHT-PSYCHOAKTIVEN CANNABINOIDEN UND VERFAHREN ZU IHRER HERSTELLUNG
COMPOSÉ D'INCLUSION CONTENANT UN CANNABINOÏDE NON PSYCHOACTIF ET SON PROCÉDÉ DE PRÉPARATION

(30) Priority: 30.05.2019 CN 201910463263
(43) Date of publication of application: 06.04.2022
(73) Proprietor: Hanyi Biotechnology (Beijing) Co., Ltd., Hujialou Chaoyang District Beijing 100020 (CN)
(72) Inventor: SUN, Wuxing, Beijing 100020 (CN); TAN, Xin, Beijing 100020 (CN); ZHANG, Jing, Beijing 100020 (CN); JIN, Qian, Beijing 100020 (CN); WANG, Shubin, Beijing 100020 (CN); XING, Junbo, Beijing 100020 (CN)
(74) Representative: Murgitroyd & Company
(86) International application number: PCT/CN2020/090347
(87) International publication number: WO 2020/238642

(56) References cited:
- WO-A1-03/070774
- WO-A1-03/070775
- WO-A1-2017/183011
- CN-A- 106 278 828
- CN-A- 106 831 353
- CN-A- 109 419 851
- CN-A- 109 568 389
- CN-A- 109 646 992
- CN-A- 110 123 876
- CYCLOLAB: "Cyclodextrin-enabled Cannabinoid Formulations", CYCLODEXTRIN NEWS, vol. 32, no. 2, 1 January 2018 (2018-01-01), pages 1-15, XP055684228, ISSN: 0951-256X
- Fang, Liang: "Inclusion Compound" In: "Pharmaceutics (3rd edition)", 31 March 2016 (2016-03-31), China Medical Science and Technology Press, CN, XP009532229, ISBN: 978-7-5067-7405-5 pages 87-89, * page 87, last paragraph to page 89, last paragraph *
- He, Yuying: "Preparation and Stability of -9-Tetrahydrocannabinol- -Cyclodextrin Inclusion Compound", Journal of International Pharmaceutical Research, no. 6, 30 June 1984 (1984-06-30), page 374, XP009532198, CN ISSN: 1674-0440, DOI: 10.13220/j.cnki.jipr.1984.06.025

## Description

### FIELD

The invention relates to the technical field of traditional Chinese medicine chemistry and preparation, in particular to an inclusion compound containing non-psychoactive cannabinoid and a method for preparation thereof

### BACKGROUND

Inclusion compound refers to a special complex formed by completely or partially encapsulating a molecule in the cavity structure of another molecule. The complex is a combination of drug molecules and inclusion material molecules through van der Waals forces. Included molecules are called included agents or guest molecules, and molecules with a cavity structure are called inclusion agents or host molecules. As a pharmaceutical preparation technology, the preparation technology of inclusion compound has developed rapidly. It plays an important role in improving the bioavailability of insoluble drugs, improving the stability of drugs, masking the taste of oily drugs, masking the bad smell or taste of drugs, regulating the release rate, and reducing the irritation and side effects of drugs.

Polyphenols in cannabis are the main active ingredients, but they have poor light stability and are easy to be oxidized. According to the types of compounds isolated at present, the main ingredients comprise the following: cannabidiol (CBD), tetrahydrocannabidiol (THC), cannabidivarin (CBDV), cannabigerol (CBG), Δ⁹-tetrahydrocannabinol (THCV), cannabinol (CBN), cannabichromene (CBC) and so on. The structural formula of the above main ingredients is as follows:

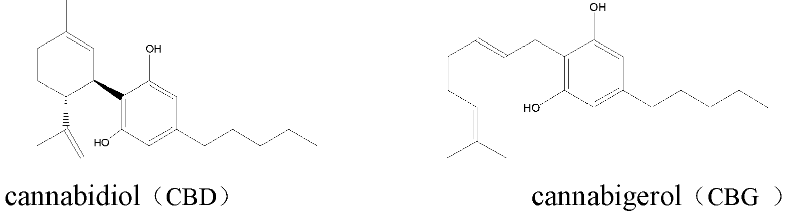

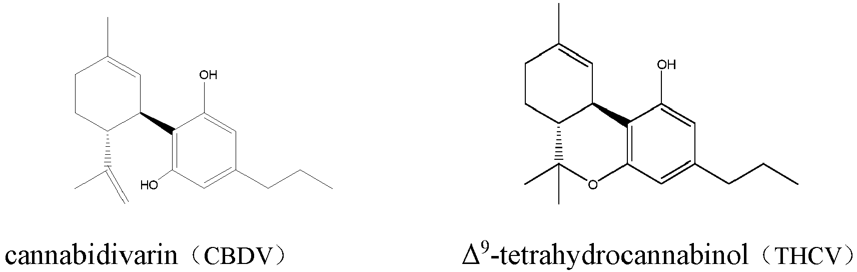

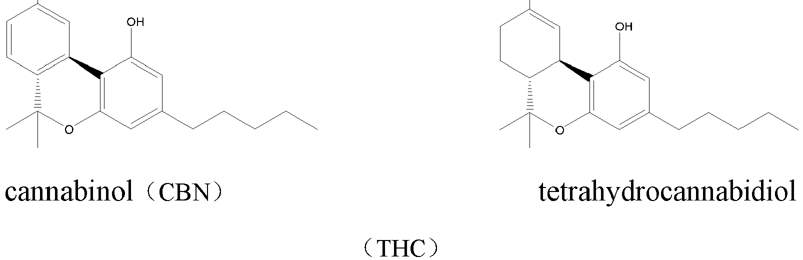

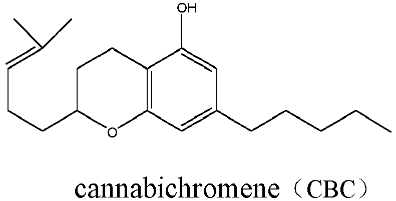

Among them, CBD, CBDV, CBG, and CBC are non-psychoactive ingredients in cannabis, namely, non-psychoactive cannabinoid; THCV, CBN, and THC are psychoactive ingredients in cannabis, namely, psychoactive cannabinoid.

The separation and purification methods for non-psychoactive cannabinoid in the prior art focus on technologies that only remove THC. For example, in patent application CN106278828A, disclosed is the use of ethanol as an extraction solvent and combining with chromatography technology to remove THC in cannabis, and only retains the effective ingredients of cannabidiol; in patent application CN107337586A, disclosed is a method for extracting cannabidiol from hemp (i.e. cannabis), the method adopts macroporous adsorption resin to adsorb cannabidiol, and further purifies cannabidiol without THC by thin-layer chromatography; in patent application CN103739585A, disclosed is a process for extracting dihydro cannabinol (CBD) from industrial hemp, the process passes chromatography purify CBD by separation. WO03/070775A1 discloses a process for making an inclusion compound containing a non-psychoactive cannabinoid. The prior art currently cannot simultaneously remove non-psychoactive cannabinoid such as CBN, THC, and THCV. While only removing THC, THCV and CBN cannot be removed at the same time, the prior art limits the development and application of cannabinoid drugs to a certain extent.

### SUMMARY

To overcome the shortcomings of the prior art, the invention aims to provide a method for the preparation of an inclusion compound containing non-psychoactive cannabinoid. The invention is defined by the appended claims.

In one aspect, the inclusion compound containing non-psychoactive cannabinoid comprises a cannabis extract and an inclusion agent, and the weight ratio of the inclusion agent to crude drug raw material of cannabis extract is 0.01-10:1 (such as 0.01:1, 0.1:1, 1:1, 3:1, 5:1, 8:1).

Further, the cannabis extract contains non-psychoactive cannabinoid and psychoactive cannabinoid.

Further, the inclusion compound essentially does not contain psychoactive cannabinoid, and "essentially does not contain" here means that the content of psychoactive cannabinoid in the inclusion compound is less than 0.01% (mass percentage) (or even less than 0.001%); Preferably, the inclusion compound does not contain psychoactive cannabinoid, i.e., the content of psychoactive cannabinoid in the inclusion compound is zero.

Further, the inclusion compound is obtained by adding the cannabis extract solution dropwise to the inclusion agent solution, stirring, separating and precipitating, and dissolving a resulting precipitate in a water-organic solvent, then separating and precipitating a resulting mixture, and drying the resulting precipitate in a vacuum.

Further, the non-psychoactive cannabinoid comprises one or a combination of two or more of cannabidiol, cannabidivarin, cannabigerol, and cannabichromene.

Further, the psychoactive cannabinoid comprises: tetrahydrocannabidiol, Δ⁹-tetrahydrocannabinol and cannabinol.

Further, the inclusion agent is cyclodextrin or a derivative thereof.

Further, the above-mentioned cyclodextrin is selected from: one or a combination of two or more of α-cyclodextrin, β-cyclodextrin, and γ-cyclodextrin in any proportion.

Further, the above-mentioned cyclodextrin derivative is selected from one or a combination of two or more of 2-hydroxypropyl cyclodextrin, 3-hydroxypropyl cyclodextrin, branched cyclodextrin, methylated cyclodextrin, and low molecular weight β-cyclodextrin Polymers (such as molecular weight 3000-6000Da), carboxymethyl cyclodextrin, cyclodextrin sulfate, cyclodextrin phosphate, cyclodextrin nitrate, cyclodextrin-epichlorohydrin cross-linked polymer and large cyclodextrin series in any proportion.

In an embodiment of the invention, the inclusion agent is β-cyclodextrin.

In another embodiment of the invention, the inclusion agent is 2-hydroxypropyl-β-cyclodextrin or 3-hydroxypropyl-β-cyclodextrin.

Further, the inclusion agent solution is an aqueous solution of the inclusion agent, wherein the weight ratio of water to crude drug raw material of the cannabis extract is 10-40:1 (such as 10:1, 15:1, 20:1, 30:1, 35:1, 40:1).

In an embodiment of the invention, the cannabis extract is a thick paste obtained by extracting the crude drug raw material of the cannabis extract, concentrating the extract, purifying, and concentrating.

Further, the crude drug material of the cannabis extract is selected from one or a combination of two or more of hemp leaves, hemp flowers, hemp roots, hemp straws, and hemp seeds in any proportion.

In an embodiment of the invention, the crude drug of the cannabis extract is cannabis flowers and/or cannabis leaves.

Further, the temperature of the dropping step is 25°C-80°C, preferably 50-70° C. (such as 50, 55, 60, 65, 70° C).

Further, the temperature of the stirring step is 25°C-80°C, preferably 50-70° C. (such as 50, 55, 60, 65, 70° C).

Further, the stirring time is 0.5-4 hours (such as 0.5, 1, 2, 3, 4 hours).

Further, in the water-organic solvent mixture, the volume ratio of water to organic solvent is 1:1-5 (such as 1:1, 1:2, 1:3, 1:4, 1:5).

Further, in the water-organic solvent mixture, the organic solvent is selected from one or a combination of two or more of ethanol, methanol, isopropanol, ethyl acetate, and acetone in any proportion.

In one embodiment of the invention, the water-organic solvent mixture is a water-ethanol mixture (such as a 50%-80% ethanol).

Further, the inclusion compound comprises non-psychoactive cannabinoid and cyclodextrin or derivatives thereof (such as β-cyclodextrin, 2-hydroxypropyl-β-cyclodextrin, 3-hydroxypropyl-β-Cyclodextrin), wherein the total content of the non-psychoactive cannabinoid is more than 50% (such as 50%-60%), and the inclusion compound does not contain psychoactive cannabinoid.

On the other hand, the invention provides a method for preparing the above-mentioned inclusion compound containing non-psychoactive cannabinoid, the method comprises the following steps:
(1) the cannabis extract was added with an organic solvent, stirred for dissolution to obtain a cannabis extract solution;
(2) the inclusion agent was weighed and dissolved in water to obtain an inclusion agent solution;
(3) the cannabis extract solution obtained in step (1) was added into the inclusion agent solution obtained in step (2) slowly, and the resulting mixture was stirred at 25°C-80°C, then cooled to room temperature, refrigerated and filtered to collect a filter cake 1;
(4) the filter cake 1 obtained in step (3) was added into the water-organic solvent, and the resulting mixture was stirred for dissolution, filtered to collect a filter cake 2;
(5) the filter cake 2 obtained in step (4) was dried in vacuum to obtain an inclusion compound containing non-psychoactive cannabinoid.

In the invention, the cannabis extract in step (1) is an extract containing cannabinoid ingredients(including psychoactive cannabinoid and non-psychoactive cannabinoid, wherein the psychoactive cannabinoid comprises one or a combination of two or more of tetrahydrocannabidiol, cannabinol, and Δ⁹-tetrahydrocannabinol.) extracted from crude drug raw material, In one embodiment of the invention, the cannabis extract in step (1) is a thick paste obtained by extracting the crude drug raw material, concentrating the extract, purifying, and concentrating.

Further, the crude drug raw material of the cannabis extract is selected from one or a combination of two or more of hemp leaves, hemp flowers, hemp roots, hemp straws, and hemp seeds in any proportion.

In an embodiment of the invention, the crude drug raw material of the cannabis extract is hemp flowers and/or hemp leaves.

In an embodiment of the invention, the above purification is purification by column chromatography.

Further, the cannabis extract in step (1) is prepared by the following steps: extracting crude drug raw material with an ethanol to obtain an extract, concentrating the extract, eluting with column chromatography, and concentrating the eluate. Among them, the concentration of ethanol can be 60-95% (v/v, such as 60%, 70%, 75%, 80%, 90%, 95%); the column packing used in column chromatography can be styrene type macroporous resin; elution can include eluting with water, 50-60% ethanol, 80-90% ethanol, and collecting 80-90% ethanol eluate.

In addition, the cannabis extract in step (1) can also be prepared by using cannabis extracts in the prior art or by using existing preparation methods.

Further, the organic solvent in step (1) is selected from one or a combination of two or more of ethanol, methanol, isopropanol, ethyl acetate, and acetone in any proportion.

Further, the inclusion agent is cyclodextrin or a derivative thereof.

Further, the above-mentioned cyclodextrin is selected from one or a combination of two or more of α-cyclodextrin, β-cyclodextrin, and γ-cyclodextrin in any proportion.

Further, the above-mentioned cyclodextrin derivative is selected from one or a combination of two or more of 2-hydroxypropyl cyclodextrin, 3-hydroxypropyl cyclodextrin, branched cyclodextrin, methylated cyclodextrin, and low molecular weight β-cyclodextrin polymers (such as molecular weight 3000-6000Da), carboxymethyl cyclodextrin, cyclodextrin sulfate, cyclodextrin phosphate, cyclodextrin nitrate, cyclodextrin-epichlorohydrin cross-linked polymer and large cyclodextrin series in any proportion.

In an embodiment of the invention, the inclusion agent is β-cyclodextrin.

In another embodiment of the invention, the inclusion agent is 2-hydroxypropyl-β-cyclodextrin or 3-hydroxypropyl-β-cyclodextrin.

Further, the weight ratio of the inclusion agent in step (2) to the crude drug raw material (such as hemp flowers and/or hemp leaves) of the cannabis extract in step (1) is 0.01-10:1 (such as 0.01: 1, 0.1:1, 1:1, 3:1, 5:1, 8:1, 10:1).

Further, the weight ratio of the water in step (2) to the crude drug raw material (such as hemp flowers and/or hemp leaves) of the cannabis extract in step (1) is 10-40:1 (such as 10:1, 15:1, 20:1, 30:1, 35:1, 40:1).

Further, the dissolution in step (3) is heating and stirring dissolution or stirring dissolution at room temperature; wherein, the heating temperature can be 50-70°C (such as 50, 55, 60, 65, 70°C).

Further, the stirring temperature in step (3) is 50-70°C (such as 50, 55, 60, 65, 70°C).

Further, the stirring time in step (3) is 0.5-4 hours (such as 0.5, 1, 2, 3, 4 hours).

Further, the refrigeration time in step (3) is 12-24 hours (such as 12, 16, 20, 24 hours).

Further, in the water-organic solvent mixture in step (4), the volume ratio of water to organic solvent is 1:1-5 (such as 1:1, 1:2, 1:3, 1:4, 1: 5).

Further, in the water-organic solvent mixture in step (4), the organic solvent is selected from one or a combination of two or more of ethanol, methanol, isopropanol, ethyl acetate, and acetone in any proportion.

In one embodiment of the invention, the water-organic solvent mixture in step (4) is a water-ethanol mixture (such as a 50%-80% ethanol).

Further, the stirring temperature in step (4) is 20-30°C (such as 20, 25, 30°C, such as room temperature).

Further, the stirring time in step (4) is 0.5-3 hours (such as 0.5, 1, 2, 3 hours).

On the other hand, the invention also provides a pharmaceutical composition comprising the above-mentioned clathrate and a pharmaceutically acceptable carrier.

On the other hand, the invention also provides an application of the aforementioned inclusion compound in the preparation of medicines, medical beauty products, and cosmetics.

On the other hand, the invention also provides an application of the method for preparing the inclusion compound in the preparation of medicines, medical beauty products, and cosmetics.

The cannabis in the invention can be cannabis from various subspecies of origin, including but not limited to cannabis produced in India, Central Asia, Bhutan, Nepal, Sikkim, China, Sativa, and Indica subspecies.

Various reagents in the invention can be selected from domestic or imported reagents that meet testing, medical, and pharmaceutical standards according to needs.

To effectively remove the psychoactive ingredients in cannabis, the invention applies the preparation technology of the inclusion compound to the removal process of psychoactive cannabinoid ingredients for the first time. Through the inclusion technology, the invention can preferentially include non-psychoactive cannabinoid such as CBD, CBDV, CBG, and CBC with high selectivity, while the selective inclusion effect of the psychoactive cannabinoid such as THCV, CBN, and THC is weak, and they are easy to be filtered and washed through water or water-organic solvent mixtures. Therefore, non-psychoactive ingredients can be retained to a great extent, and the stability of the non-psychoactive ingredients will be greatly improved after washing. The obtained inclusion compound is beneficial to the forming of the follow-up preparation.

The invention provides a preparation method for enriching and purifying non-psychoactive cannabinoid. The raw materials, reagents, and instruments used are cheap and easy to obtain, and the obtained inclusion compound can be directly used as a preparation intermediate, the obtained inclusion compound is beneficial to the forming of the follow-up preparation, the operation and method adopted are simple, easy to realize industrialization, and can be used in the field of medicine.

### BRIEF DESCRIPTION OF THE DRAWINGS

FIG. 1 is a liquid chromatogram of an ethanol solution in step (2) in Example 1 of the invention.
FIG.2 is a liquid chromatogram of filtrate 1 in step (5) in Example 1 of the invention.
FIG.3 is a liquid chromatogram of filtrate 2 in step (5) in Example 1 of the invention.
FIG.4 is a liquid chromatogram of the vacuum-dried product of the filter cake 2 in step (5) in Example 1 of the invention.

### DETAILED DESCRIPTION

Unless otherwise defined, all technical and scientific terms used herein have the same meaning as commonly understood by those skilled in the field of the invention, such as the following abbreviations and their corresponding substances appearing in the invention are:
- CBDV: cannabidivarin
- CBD: cannabidiol
- CBG: cannabigerol
- THCV: Δ⁹-tetrahydrocannabinol
- THC: tetrahydrocannabidiol
- CBC: cannabichromene

In the invention, the term "cannabinoid" refers to secondary metabolites unique to the cannabis plant containing the molecular structure of alkyl and monoterpenoid groups, for example, CBD, THC, CBDV, CBG, THCV, CBN, CBC, etc. (As described in "Chen X, Yang M, Guo HY (2011). Research advances in cannabinoids of Cannabis sativa. Chin Bull Bot 46, 197-205"). Psychoactive substances refer to chemical substances affecting human emotions and behaviors, changing the state of consciousness, and causing dependence. In the invention, the term "psychoactive cannabinoid" refers to cannabinoid with the above-mentioned psychoactive, such as THC, CBN, THCV, etc., while "non-psychoactive cannabinoid" refers to cannabinoid that do not have the above-mentioned psychoactive, such as CBD, CBDV, CBG, CBC, etc.

The term "crude drug" refers to pure natural traditional Chinese medicine materials without processing or simple processing of plant, animal, and mineral. In the invention, the crude drug of the cannabis extract is cannabis medicinal material, and the medicinal parts of cannabis medicinal material include leaves, flowers, roots, stalks, seeds, etc.

In order to be able to understand the technical content of the invention more clearly, the following examples are specifically described in detail, the purpose of the following examples is only to better understand the content of the invention and not to limit the protection scope of the invention.

### Example 1

The preparation steps of the inclusion compound containing non-psychoactive cannabinoid are as follows:
(1) 10 kg medicinal material of hemp flower and leaf was added with 100 kg of 75% ethanol, and the resulting mixture was stirred and extracted at room temperature for 1 hour, extracted for 2 times, and then the resulting extracts were combined and concentrated under reduced pressure to a density of 1.014 (50°C), and purified by a styrene-type macroporous resin column, the sample after loaded was left standing for 30 minutes, and then eluted with 5 times column volume of purified water, 3 times column volume of 55% ethanol, 6 times volume of 85% ethanol in turn, then the 85% ethanol eluate was collected and concentrated under reduced pressure to obtain a thick paste;
(2) the thick paste obtained in step (1) was added with 95% (v/v, the same below) ethanol, stirred for dissolution to obtain an ethanol solution;
(3) 50kg β-cyclodextrin was weighed and added into 300 kg of purified water, and then the resulting mixture was heated and stirred for dissolution at 60°C to obtain an inclusion agent solution;
(4) the ethanol solution obtained in step (2) was slowly added into the inclusion agent solution obtained in step (3), and the resulting mixture was stirred at 60°C for 4 hours until the addition was completed, after cooling to room temperature, the resulting mixture was refrigerated for 18 hours;
(5) the refrigerated liquid obtained in step (4) was filtered to collect a filtrate 1 and a filter cake 1, the filter cake 1 was added into 65% ethanol, and the resulting mixture was stirred for dissolution at 30°C for 1 hour, and then filtered to collect a filtrate 2 and a filter cake 2, the collected filter cake 2 was dried in a vacuum environment to obtain the inclusion compound of non-psychoactive cannabinoids;
(6) the content of CBDV, CBD, CBG, THCV and THC in the vacuum dried product of the ethanol solution obtained in step (2), filtrate 1, filtrate 2 and filter cake 2 obtained in step (5) were detected respectively .

The sample detection and analysis methods are as follows:
Chromatographic conditions and system applicability test: octadecyl silane bonded silica gel was used as a filler, 0.1% formic acid aqueous solution as mobile phase A, 0.1% formic acid in acetonitrile as mobile phase B, the elution procedure is as follows, the detection wavelength was 220 nm, and the number of theoretical plates calculated by CBD was not less than 5000.

When the elution time was 0-6 minutes, the volume concentration of the mobile phase: mobile phase A: 30%, and mobile phase B: balance; when the elution time was 6-12 minutes, the volume concentration of the mobile phase: mobile phase A: 30% to 23% in a gradient, and mobile phase B: balance; when the elution time was 12-22 minutes, the volume concentration of the mobile phase: mobile phase A: 23%, and mobile phase B: balance; when the elution time was 22-22.2 minutes, the volume concentration of the mobile phase: mobile phase A: 23% to 30% in a gradient, and mobile phase B: balance; and when the elution time was 22.2-26 minutes, the volume concentration of the mobile phase: mobile phase A:30%, and mobile phase B: balance.

Preparation of reference solution: CBD reference substance was precisely weighed and added with methanol to obtain a reference substance solution containing 0.15mg per 1ml; CBDV reference substance was precisely weighed and added with methanol to obtain a reference substance solution containing 0.01mg per 1ml; THCV reference substance was precisely weighed and added with methanol to obtain a reference substance solution containing 0.01mg per 1ml; CBG reference substance was precisely weighed and added with methanol to obtain a reference substance solution containing 0.01mg per 1ml; THC reference substance was precisely weighed and added with methanol to obtain a reference substance solution containing 0.01mg per 1ml; CBC reference substance was precisely weighed and added with methanol to obtain a reference substance solution containing O.Olmg per 1ml.

Preparation of test solution: ①1.5mL the obtained ethanol solution or filtrate was measured and added with methanol to make the volume to 5mL, then filtered with a microporous membrane (0.45 µ m) to obtain an additional filtrate; ②2.5g the resulting filter cake was measured and added with methanol to make the volume to 50mL, then filtered with a microporous membrane (0.45µm) to obtain an additional filtrate.

Determination method: the reference solution and the test solution were precisely absorbed 10µ L each and injected into the liquid chromatography for determination.

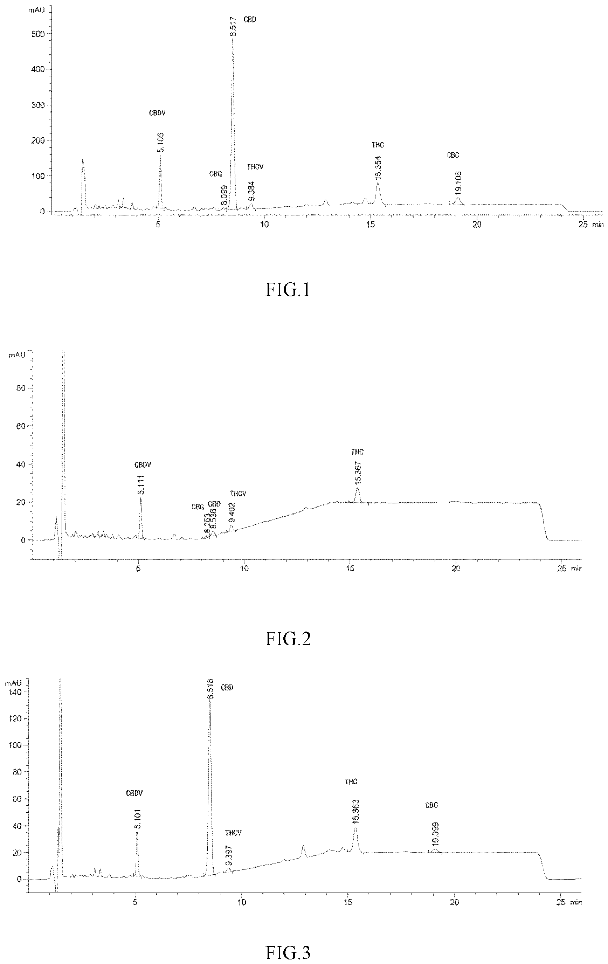

The detection chromatogram of the ethanol solution is shown in FIG. 1, where the retention time and corresponding substances are as follows: 5.105 is CBDV, 8.099 is CBG, 8.517 is CBD, 8.384 is THCV, 15.354 is THC, and 19.106 is CBC.

The detection chromatogram of filtrate 1 is shown in FIG. 2, where the retention time and corresponding substances are as follows: 5.111 is CBDV, 8.253 is CBG, 8.536 is CBD, 9.402 is THCV, and 15.367 is THC.

The detection chromatogram of filtrate 2 is shown in FIG. 3, where the retention time and corresponding substances are as follows: 5.101 is CBDV, 8.518 is CBD, 9.3976 is THCV, 15.363 is THC, and 19.099 is CBC.

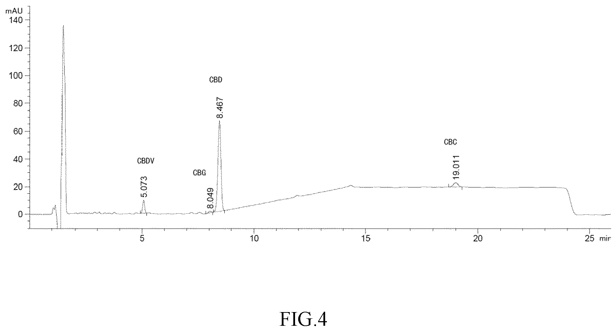

The detection chromatogram of the dried substance of filter cake 2 is shown in FIG. 4, in which the retention time and corresponding substances are as follows: 5.073 is CBDV, 8.049 is CBG, 8.467 is CBD, and 19.011 is CBC.

Results: the inclusion compound containing non-psychoactive cannabinoids prepared in this example, the total content of cannabinoids was 55.5%, and THCV and THC were not detected.

### Example 2

The preparation steps of the inclusion compound containing non-psychoactive cannabinoid are as follows:
(1) 10 kg medicinal material of hemp flower and leaf was added with 100 kg of 95% ethanol, and the resulting mixture was stirred and extracted at room temperature for 0.5 hour, extracted for 4 times, and then the resulting extracts were combined and concentrated under reduced pressure to a density of 1.014 (50°C), and purified by a styrene-type macroporous resin column, the sample after loaded was left standing for 30 minutes, and then eluted with 5 times column volume of purified water, 3 times column volume of 55% ethanol, 6 times volume of 85% ethanol in turn, then the 85% ethanol eluate was collected and concentrated under reduced pressure to obtain a thick paste;
(2) the thick paste obtained in step (1) was added with 95% (v/v, the same below) isopropanol, stirred for dissolution to obtain an isopropanol solution;
(3) 10kg 2-hydroxypropyl-β-cyclodextrin was weighed and added into 100 kg of purified water, and then the resulting mixture was heated and stirred for dissolution at 70°C to obtain an inclusion agent solution;
(4) the isopropanol solution obtained in step (2) was slowly added into the inclusion agent solution obtained in step (3), and the resulting mixture was stirred at 70°C for 0.5 hours until the addition was completed, after cooling to room temperature, the resulting mixture was refrigerated for 12 hours;
(5) the refrigerated liquid obtained in step (4) was filtered to collect a filtrate 1 and a filter cake 1, the filter cake 1 was added into 80% isopropanol, and the resulting mixture was stirred for dissolution at 20°C for 3 hour, and then filtered to collect a filtrate 2 and a filter cake 2, the collected filter cake 2 was dried in a vacuum environment to obtain the inclusion compound of non-psychoactive cannabinoids;
(6) the content of CBDV, CBD, CBG, THCV and THC in the vacuum dried product of the filter cake 2 obtained in step (5) was detected, and the detection methods and steps refer to the relevant methods and steps in Example 1.

Results: the inclusion compound containing non-psychoactive cannabinoids prepared in this example, the total content of cannabinoids was 50.5%, and THCV and THC were not detected.

### Example 3

The preparation steps of the inclusion compound containing non-psychoactive cannabinoid are as follows:
(1) 10 kg medicinal material of hemp flower and leaf was added with 150 kg of 75% ethanol, and the resulting mixture was stirred and extracted at room temperature for 1 hour, extracted for 3 times, and then the resulting extracts were combined and concentrated under reduced pressure to a density of 1.014 (50°C), and purified by a styrene-type macroporous resin column, the sample after loaded was left standing for 30 minutes, and then eluted with 5 times column volume of purified water, 3 times column volume of 55% ethanol, 6 times volume of 85% ethanol in turn, then the 85% ethanol eluate was collected and concentrated under reduced pressure to obtain a thick paste;
(2) the thick paste obtained in step (1) was added with an appropriate amount of acetone, stirred for dissolution to obtain an acetone solution;
(3) 100 kg 3-hydroxypropyl-β-cyclodextrin was weighed and added into 400 kg of purified water, and then the resulting mixture was heated and stirred for dissolution at 50°C to obtain an inclusion agent solution;
(4) the acetone solution obtained in step (2) was slowly added into the inclusion agent solution obtained in step (3), and the resulting mixture was stirred at 50°C for 2 hours until the addition was completed, after cooling to room temperature, the resulting mixture was refrigerated for 24 hours;
(5) the refrigerated liquid obtained in step (4) was filtered to collect a filtrate 1 and a filter cake 1, the filter cake 1 was added into 50% acetone, and the resulting mixture was stirred for dissolution at 25°C for 0.5 hour, and then filtered to collect a filtrate 2 and a filter cake 2, the collected filter cake 2 was dried in a vacuum environment to obtain the inclusion compound of non-psychoactive cannabinoids;
(6) the content of CBDV, CBD, CBG, THCV and THC in the vacuum dried product of the filter cake 2 obtained in step (5) was detected, and the detection methods and steps refer to the relevant methods and steps in Example 1.

Results: the inclusion compound containing non-psychoactive cannabinoids prepared in this example, the total content of cannabinoids was 52.4%, and THCV and THC were not detected.

### Example 4

The preparation steps of the inclusion compound containing non-psychoactive cannabinoid are as follows:
(1) 10kg raw medicinal material of hemp flower and leaf was taken, and the thick paste of cannabis extract was prepared by referring to the experimental steps (1)-(7) of Example 1 of the Chinese application CN109568389A.
(2) the thick paste obtained in step (1) was added with an appropriate amount of ethanol, stirred for dissolution to obtain an ethanol solution;
(3) 30 kg β-cyclodextrin was weighed and added into 200 kg of purified water, and then the resulting mixture was heated and stirred for dissolution at 65°C to obtain an inclusion agent solution;
(4) the acetone solution obtained in step (2) was slowly added into the inclusion agent solution obtained in step (3), and the resulting mixture was stirred at 65°C for 4 hours until the addition was completed, after cooling to room temperature, the resulting mixture was refrigerated for 20 hours;
(5) the refrigerated liquid obtained in step (4) was filtered to collect a filtrate 1 and a filter cake 1, the filter cake 1 was added into 80% ethanol, and the resulting mixture was stirred for dissolution at 25°C for 1.5 hour, and then filtered to collect a filtrate 2 and a filter cake 2, the collected filter cake 2 was dried in a vacuum environment to obtain the inclusion compound of non-psychoactive cannabinoids;
(6) the content of CBDV, CBD, CBG, THCV and THC in the vacuum dried product of the filter cake 2 obtained in step (5) was detected, and the detection methods and steps refer to the relevant methods and steps in Example 1.

Results: the inclusion compound containing non-psychoactive cannabinoids prepared in this example, the total content of cannabinoids was 65.8 %, and THCV and THC were not detected. According to the Chinese application CN109568389A, the cannabis extract obtained in Example 1 contains 4.1% THCV. It can be seen that the hydrate prepared by the method of the invention can retain non-psychotropic cannabinoids with high selectivity and eliminate psychoactive cannabinoids.

## Claims

1. A method for preparing an inclusion compound, wherein the method comprises the following steps:
(1) dissolving a cannabis extract, said cannabis extract containing non-psychoactive cannabinoid and psychoactive cannabinoid, with an organic solvent to obtain a cannabis extract solution; (2) weighing an inclusion agent and dissolving in water to obtain an inclusion agent solution; (3) adding the solution obtained in step (1) dropwise to the inclusion agent solution obtained in step (2), stirring at 50- 70°C, and then refrigerating after cooling to room temperature, filtering to obtain a filter cake 1; (4) adding the water-organic solvent into the filter cake 1 obtained in step (3), stirring, dissolving, and filtering to obtain a filter cake 2; (5) vacuum drying the filter cake 2 obtained in step 4 to obtain the inclusion compound containing non- psychoactive cannabinoid; wherein
the non-psychoactive cannabinoid comprises one or a combination of two or more of cannabidiol, cannabidivarin, cannabigerol, and cannabichromene and the psychoactive cannabinoid comprises tetrahydrocannabidiol, Δ⁹-tetrahydrocannabinol and cannabinol;
the inclusion agent in step (3) is cyclodextrin or a derivative thereof;
the stirring time in step (3) is 0.5-4 hours;the refrigeration time in step (3) is 12-24 hours; in the water-organic solvent in step (4), the volume ratio of water to organic solvent is 1:1-5 ; the stirring temperature in step (4) is 20-30°C; the stirring time in step (4) is 0.5-3 hours;
the organic solvent in steps (1) and (4) is independently selected from one or a combination of two or more of ethanol, methanol, isopropanol, ethyl acetate, and acetone in any proportion.

2. The preparation method according to claim 1, wherein the cannabis extract in step (1) is a thick paste obtained by extracting a crude drug raw material, concentrating the extract, purifying, and concentrating; the crude drug raw material is selected from one or a combination of two or more of hemp leaves, hemp flowers, hemp roots, hemp straws and hemp seeds in any proportion.

3. The preparation method according to claim 2, wherein the crude drug raw material is selected from hemp flowers and/or hemp leaves.

4. The preparation method according to claim 1, wherein the cyclodextrin is selected from one or a combination of two or more of α- cyclodextrin, β - cyclodextrin, and γ- cyclodextrin in any proportion; the cyclodextrin derivative is selected from one or a combination of two or more of 2-hydroxypropyl cyclodextrin, 3 -hydroxypropyl cyclodextrin, branched cyclodextrin, methylated cyclodextrin, and low molecularweight β-cyclodextrin polymers, carboxymethyl cyclodextrin, cyclodextrin sulfate, cyclodextrin phosphate, cyclodextrin nitrate, cyclodextrin-epichlorohydrin cross- linked polymer and large cyclodextrin series in any proportion.

5. The preparation method according to claim 2-4, wherein the weight ratio of the inclusion agent in step (2) to the crude drug raw material of the cannabis extract in step (1) is 0.01- 10:1; the weight ratio of the water in step (2) to the crude drug raw material of the cannabis extract in step (1) is 10-40:1.

## Patentansprüche

1. Ein Verfahren zum Herstellen einer Einschlussverbindung, wobei das Verfahren die folgenden Schritte beinhaltet:
(1) Auflösen eines Cannabisextrakts, wobei der Cannabisextrakt nicht psychoaktives Cannabinoid und psychoaktives Cannabinoid enthält, mit einem organischen Lösungsmittel, um eine Cannabisextraktlösung zu erhalten; (2) Wiegen eines Einschlussmittels und Auflösen in Wasser, um eine Einschlussmittellösung zu erhalten; (3) tröpfchenweises Zugeben der in Schritt (1) erhaltenen Lösung zu der in Schritt (2) erhaltenen Einschlussmittellösung, Rühren bei 50-70 °C und dann Kühlen nach Abkühlen auf Raumtemperatur, Filtern, um einen Filterkuchen 1 zu erhalten; (4) Zugeben des Wasser-organischen Lösungsmittels in den in Schritt (3) erhaltenen Filterkuchen 1, Rühren, Auflösen und Filtern, um einen Filterkuchen 2 zu erhalten; (5) Vakuumtrocknen des in Schritt 4 erhaltenen Filterkuchens 2, um die Einschlussverbindung zu erhalten, die nicht psychoaktives Cannabinoid enthält; wobei das nicht psychoaktive Cannabinoid eines oder eine Kombination von zwei oder mehr von Cannabidiol, Cannabidivarin, Cannabigerol und Cannabichromen beinhaltet und das psychoaktive Cannabinoid Tetrahydrocannabidiol, Δ⁹-Tetrahydrocannabinol und Cannabinol beinhaltet;
das Einschlussmittel in Schritt (3) Cyclodextrin oder ein Derivat davon ist;
die Rührdauer in Schritt (3) 0,5-4 Stunden beträgt; die Kühldauer in Schritt (3) 12-24 Stunden beträgt; das Volumenverhältnis von Wasser zu organischem Lösungsmittel in dem Wasser-organischen Lösungsmittel in Schritt (4) 1 : 1-5 beträgt; die Rührtemperatur in Schritt (4) 20-30 °C beträgt; die Rührdauer in Schritt (4) 0,5-3 Stunden beträgt;
das organische Lösungsmittel in den Schritten (1) und (4) unabhängig aus einem oder einer Kombination von zwei oder mehr von Ethanol, Methanol, Isopropanol, Ethylacetat und Aceton in beliebiger Proportion ausgewählt ist.

2. Herstellungsverfahren gemäß Anspruch 1, wobei der Cannabisextrakt in Schritt (1) eine dicke Paste ist, die durch das Extrahieren eines Drogenrohmaterials, Konzentrieren des Extrakts, Reinigen und Konzentrieren erhalten wird; das Drogenrohmaterial aus einem oder einer Kombination von zwei oder mehr von Hanfblättern, Hanfblüten, Hanfwurzeln, Hanfstrohhalmen und Hanfsamen in beliebiger Proportion ausgewählt ist.

3. Herstellungsverfahren gemäß Anspruch 2, wobei das Drogenrohmaterial aus Hanfblüten und/oder Hanfblättern ausgewählt ist.

4. Herstellungsverfahren gemäß Anspruch 1, wobei das Cyclodextrin aus einem oder einer Kombination von zwei oder mehr von α-Cyclodextrin, β-Cyclodextrin und γ-Cyclodextrin in beliebiger Proportion ausgewählt ist; das Cyclodextrinderivat aus einem oder einer Kombination von zwei oder mehr von 2-Hydroxypropylcyclodextrin, 3-Hydroxypropylcyclodextrin, verzweigtem Cyclodextrin, methyliertem Cyclodextrin und niedermolekularen β-Cyclodextrinpolymeren, Carboxymethylcyclodextrin, Cyclodextrinsulfat, Cyclodextrinphosphat, Cyclodextrinnitrat, cyclodextrinepichlorhydrinvernetztem Polymer und Reihen großen Cyclodextrins in beliebiger Proportion ausgewählt ist.

5. Herstellungsverfahren gemäß Anspruch 2-4, wobei das Gewichtsverhältnis des Einschlussmittels in Schritt (2) zu dem Drogenrohmaterial des Cannabisextrakts in Schritt (1) 0,01-10 : 1 beträgt; das Gewichtsverhältnis des Wassers in Schritt (2) zu dem Drogenrohmaterial des Cannabisextrakts in Schritt (1) 10-40 : 1 beträgt.

## Revendications

1. Un procédé pour la préparation d'un composé d'inclusion, le procédé comprenant les étapes suivantes :
(1) la dissolution d'un extrait de cannabis, ledit extrait de cannabis contenant du cannabinoïde non psychoactif et du cannabinoïde psychoactif, avec un solvant organique afin d'obtenir une solution d'extrait de cannabis ; (2) le pesage d'un agent d'inclusion et la dissolution dans de l'eau afin d'obtenir une solution d'agent d'inclusion ; (3) l'ajout de la solution obtenue à l'étape (1) goutte à goutte à la solution d'agent d'inclusion obtenue à l'étape (2), l'agitation à 50 à 70 °C, et ensuite la réfrigération après refroidissement jusqu'à température ambiante, la filtration afin d'obtenir un gâteau de filtration 1 ; (4) l'ajout du solvant organique-eau dans le gâteau de filtration 1 obtenu à l'étape (3), l'agitation, la dissolution, et la filtration afin d'obtenir un gâteau de filtration 2 ; (5) le séchage sous vide du gâteau de filtration 2 obtenu à l'étape 4 afin d'obtenir le composé d'inclusion contenant du cannabinoïde non psychoactif ; dans lequel
le cannabinoïde non psychoactif comprend un élément ou une combinaison de deux éléments ou plus parmi le cannabidiol, la cannabidivarine, le cannabigérol, et le cannabichromène et le cannabinoïde psychoactif comprend du tétrahydrocannabidiol, du Δ⁹-tétrahydrocannabinol et du cannabinol ;
l'agent d'inclusion à l'étape (3) est une cyclodextrine ou un dérivé de celle-ci ;
le temps d'agitation à l'étape (3) est de 0,5 à 4 heures ; le temps de réfrigération à l'étape (3) est de 12 à 24 heures ; dans le solvant organique-eau à l'étape (4), le rapport en volume de l'eau au solvant organique est de 1/1 à 5 ; la température d'agitation à l'étape (4) est de 20 à 30 °C ; le temps d'agitation à l'étape (4) est de 0,5 à 3 heures ;
le solvant organique aux étapes (1) et (4) est sélectionné indépendamment parmi un élément ou une combinaison de deux éléments ou plus parmi l'éthanol, le méthanol, l'isopropanol, l'acétate d'éthyle, et l'acétone dans n'importe quelle proportion.

2. Le procédé de préparation selon la revendication 1, dans lequel l'extrait de cannabis à l'étape (1) est une pâte épaisse obtenue par extraction d'une matière première brute, concentration de l'extrait, purification, et concentration ; la matière première brute est sélectionnée parmi un élément ou une combinaison de deux éléments ou plus parmi les feuilles de chanvre, les fleurs de chanvre, les racines de chanvre, les pailles de chanvre et les graines de chanvre dans n'importe quelle proportion.

3. Le procédé de préparation selon la revendication 2, dans lequel la matière première brute est sélectionnée parmi les fleurs de chanvre et/ou les feuilles de chanvre.

4. Le procédé de préparation selon la revendication 1, dans lequel la cyclodextrine est sélectionnée parmi un élément ou une combinaison de deux éléments ou plus parmi l'α-cyclodextrine, la β-cyclodextrine, et la γ-cydodextrine dans n'importe quelle proportion ; le dérivé de cyclodextrine est sélectionné parmi un élément ou une combinaison de deux éléments ou plus parmi la cyclodextrine 2-hydroxypropyle, la cyclodextrine 3-hydroxypropyle, une cyclodextrine ramifiée, une cyclodextrine méthylée, et des polymères de β-cyclodextrine de faible poids moléculaire, la cyclodextrine de carboxyméthyle, le sulfate de cyclodextrine, le phosphate de cyclodextrine, le nitrate de cyclodextrine, un polymère réticulé de cyclodextrine-épichlorhydrine et des séries de larges cyclodextrines dans n'importe quelle proportion.

5. Le procédé de préparation selon les revendications 2 à 4, dans lequel le rapport en poids de l'agent d'inclusion à l'étape (2) à la matière première brute de l'extrait de cannabis à l'étape (1) est de 0,01 à 10/1 ; le rapport en poids de l'eau à l'étape (2) à la matière première brute de l'extrait de cannabis à l'étape (1) est de 10 à 40/1.
